# EUROPEAN PATENT SPECIFICATION

(11) **EP 2 035 577 B1**
(45) Date of publication and mention of the grant of the patent: **03.07.2013**
(21) Application number: 07701052.8
(22) Date of filing: 24.01.2007
(51) Int. Cl.: C12Q 1/68

(54) **METHOD FOR DIAGNOSING HEPATOCELLULAR CARCINOMA**
VERFAHREN ZUR DIAGNOSE VON LEBERZELLENKARZINOMEN
PROCÉDÉ DE DIAGNOSTIC DE CARCINOME HÉPATOCELLULAIRE

(30) Priority: 07.11.2006 KR 20060109483
(43) Date of publication of application: 18.03.2009
(73) Proprietor: Chonbuk National University Industrial Cooperation Foundation, Jeonju-si, Jeollabuk-do 561-756 (KR)
(72) Inventor: KIM, Dae-Ghon, Jeollabuk-do 561-740 (KR); YU, Gyung-Ran, Jeollabuk-do 561-740 (KR)
(74) Representative: Bassil, Nicholas Charles
(86) International application number: PCT/KR2007/000411
(87) International publication number: WO 2008/056854

(56) References cited:
- WO-A2-03/061564
- US-A1- 2003 108 553
- ALAKURTTI K ET AL.: "Loss of lysosomal association of cystatin B proteins representing progressive myoclonus epilepsy, EPM1, mutations" EUROPEAN JOURNAL OF HUMAN GENETICS, vol. 13, February 2005 (2005-02), pages 208-215, XP002547861
- LEE M-J ET AL.: "Identification of cystatin B as a potential serum marker in hepatocellular carcinoma" CLINICAL CANCER RESEARCH, vol. 14, no. 4, 15 February 2008 (2008-02-15), pages 1080-1089, XP002547862
- PENNACCHIO L.A. ET AL.: 'Mutations in the gene encoding cystatin B in progressive myoclonus epilepsy (EPM1)' SCIENCE vol. 271, no. 5256, 22 March 1996, pages 1731 - 1734, XP002192539
- CHU S.-C. ET AL.: 'Increased cystatin C serum concentrations in patients with hepatic diseases of various severities' CLINICA CHIMICA ACTA vol. 41, 2004, pages 133 - 138, XP008128935
- TAKESHI SHIRAISHI ET AL.: 'Identification of cystatin B in human esophageal carcinoma, using differential displays in which the gene expression is related to lymph-node metastasis' INTERNATIONAL JOURNAL OF CANCER vol. 79, no. 2, 06 December 1998, pages 175 - 178, XP008128909

<110> INDUSTRIAL COOPERATION FOUNDATION CHONBUK NATIONAL UNIVERSITY
<120> A diagnostic composition for hepatocellular carcinoma, a diagnostic kit comprising it and diagnostic methods of hepatocellular carcinoma
<130> PA9608-595/KR
<160> 4
<170> KopatentIn 1.71
<210> 1
   <211> 21
   <212> DNA
   <213> Artificial sequence
<220>
   <223> a forward primer1 for RT-PCR
<400> 1
   gtcgccgcca agatgatgtg c 21
<210> 2
   <211> 20
   <212> DNA
   <213> Artificial sequence
<220>
   <223> a forward primer2 for RT-PCR
<400> 2
   tgtcattcaa gagccaggtg 20
<210> 3
   <211> 21
   <212> DNA
   <213> Artificial sequence
<220>
   <223> a backward primer1 for RT-PCR
<400> 3
   gaaataggtc agctcatcat g 21
<210> 4
   <211> 20
   <212> DNA
   <213> Artificial sequence
<220>
   <223> a backward primer2 for RT-PCR
<400> 4
   gctctggtag acggaggatg 20

## Description

### [Technical Field]

The present invention relates to a method for diagnosing hepatocellular carcinoma by measuring cystatin B (CSTB, stefin B) present in the tissue and body fluid of a patient with hepatocellular carcinoma.

### [Background Art]

There are various methods for diagnosing cancers using tumor markers. Most of them are performed by a blood test using an antibody, and some of them were performed using tissue extract fluid, urine, stool, or the like. The tumor markers vary according to the regions, in which cancers occur, but there is no tumor marker to detect all kinds of human cancers. Further, there are many cancers in that tumor markers are not found. Therefore, in order to develop better cancer diagnosis using tumor markers, studies have been made on diagnosis using a monoclonal antibody, a laser beam, a radioisotope, a chemiluminescence probe, an automatic analyzer or the like.

There are specific tumor markers that are only produced by specific cancer cells in the human body. Therefore, an elevated level of the tumor marker makes it possible to diagnose a cancer occurring in a specific organ, which is referred to as "high organ specificity". Examples of the tumor markers belonging to the above mentioned groups include a prostate and prostate cancer-specific antigen (PSA), a placenta, an embryonic cell and human chorionic gonadotropin (hCG), a neuroendocrine cell and a neuron-specific enolase (NSE), endocrine glands and various hormone, a neurilemoma and catecholamine (VMA), a hepatic cell, an embryonic cell and alpha fetoprotein (AFP). However, in many cases, a tumor marker is produced by several organs. Accordingly, even if the level of the specific marker is elevated, it is difficult to determine the organ where the cancer occurs. For example, the tumor marker such as CEA and CA19-9 is produced by cancer cells in various organs including stomach, colon, pancreas, and lung. Thus, even if the tumor marker is present in serum with a large amount, it is impossible to determine the organ where the cancer occurs.

Nevertheless, tumor markers are usefully employed in cancer diagnosis, and tumor markers vary widely depending on the kinds of cancers, since early diagnosis is very important, and a patient at the earliest stage of cancer is not aware of any symptom. Further, there is no tumor marker capable of detecting all kinds of cancers by a one-step test, and many cancers produce no effective tumor marker. Accordingly, many studies have attempted to search better tumor marker.

Hepatocellular carcinoma is a primary malignant tumor, which occurs in the liver. The cancer cells from elsewhere in the body metastasize to the liver, so as to develop metastatic hepatic carcinoma. 90% or more of total liver cancers are hepatocellular carcinoma. Even after curative treatment of hepatocellular carcinoma, the recurrence rate is from 40 to 80%. In most of cases, tumors recur in the liver, but they may recur in the lung and lymph node, or the inner surface of the abdominal wall and mediastinum. The ratio of the number male to female patients hepatocellular carcinoma is 4 to 1, and these tumors mostly occur in the middle-aged and more elderly patients.

Hepatocellular carcinoma is caused by hepatitis B virus, hepatitis C virus, alcoholic liver disease, metabolic liver disease, and other toxins. In particular, 65 to 80% of patients with Hepatocellular carcinoma in Korea are known to have Hepatitis B antigen carrier.

A patient at an early stage of hepatocellular carcinoma has no symptom, and if the patient experiences some symptoms, he or she is already at an advanced stage. The symptoms are mainly fatigue, abdominal pain or distension, and loss of appetite. The specific symptom is palpable abdominal massiveness at precordial area, which indicates that the cancer has significantly advanced. When the mass of hepatocellular carcinoma may burst in abdominal cavity, the patient has abrupt abdominal pain and distension, hypotension or shock, or the like.

The patients with hepatocellular carcinoma may die of tumor progression, and they may die of cirrhosis accompanied. Therefore, a treatment to prevent the development of hepatocellular carcinoma and cirrhosis simultaneously is needed. When the cancer is early diagnosed, and thus the surgery is performed, it can be completely cured. On the other hand, in the case of patients with the significantly advanced cancer, bad liver function, or metastatic cancer, therapies such as percutaneous ethanol injection, transarterial chemoembolization, and radiofrequency ablation are performed instead of surgery.

Since the prognosis of hepatocellular carcinoma is not good, whereby its prevention and early diagnosis is important. In order to prevent and diagnose early the disease, inoculation of hepatitis B vaccine has to be performed. Further, patients with chronic hepatitis or cirrhosis have to take a medical examination once every three to six months.

Recently, a tumor suppressor gene such as p53, -catenin, and Axinl, or mutation of oncogene was found in tumor tissue and there is a report that such genes may be involved in occurrence of liver cancer. However, the mutation frequency of the genes is very low, whereby it is difficult to determine a correlation between the mutation of the genes and the progression of liver cancer. Accordingly, molecular biological studies on the cause and progression of liver cancer are still challenges to be solved.

Hepatocellular carcinoma occurring in Korea is generally developed with previously occurred cirrhosis, and the liver tissue accompanied by cirrhosis has been known to include an intermediate stage between the nonneoplastic regenerating nodule as a precursor lesion and the malignant hepatocellular carcinoma. The nodule lesion is said to be a dysplastic nodule, which can be classified into a low grade dysplastic nodule and a high grade dysplastic nodule according to its grade. Small hepatocellular carcinomas are frequently found in the high grade dysplastic nodule. Therefore, the high grade dysplastic nodule has been regarded as a precancerous stage of hepatocellular carcinoma. The precursor lesion for liver cancer can be histologically classified into early hepatocellular carcinoma, carcinoma in situ, or the like according to pathologists. The precancerous stage of liver cancer is still controversial issue, and molecular mechanism underlying the development of hepatocellular carcinoma from the precancerous stage has not been clearly revealed yet. Hepatocellular carcinoma is histopathologically classified into four grades according to Edmondson grade. The differentiation degree and size of tumor cells is noted to represent morphological changes, which indicates that the progression of liver cancer is stepwise, but the molecular biological mechanism thereof has not been revealed yet.

A DNA microarray technology has been recently introduced to enable the comprehensive analysis of gene expression, and is a new genomic technology employed in oncology studies. Some researchers have tried to demonstrate the development of liver cancer using molecular expression profiles, but large gene family at each stage according to the progression of hepatocellular carcinoma at early and late stage has not been examined yet, and the examination will provide an important information for understanding the development of hepatocellular carcinoma at an early stage and the invasiveness and metastasis of hepatocellular carcinoma at a later stage.

A representative molecular marker for hepatocellular carcinoma, AFP, is a glycoprotein that is abnormally produced when hepatic cells are transformed to be de-differentiated into cancerous cells, and used for discovery and observation in treatment of primary liver cancer, hepatitis, cirrhosis, and yolk sac tumor. The AFP is produced in fetal liver or yolk sac. Its concentration reaches to a peak at the week 13, and thereafter sharply decreased. Its concentration is very low in normal adult (7 to 10 ng/mL or less), but the increase in AFP level means that serious cancerous cells increase, in particular, in patient with liver cell associated cancer, 50 to 70% or more of the increase in AFP level is observed. If the AFP level is 7 to 15 ng or less per 1 ml of blood, it is considered to be negative.

In the case of liver cancer, hepatitis, and cirrhosis, which occur with liver cancer simultaneously, the tumor marker, AFP, may be produced and its amount has been known to increase to around 500 ng/mL. Further, in obstetrics and gynecology, the AFP increase in blood or amniotic fluid of the pregnant can be an index for detecting congenital defects such as anencephaly, spina bifida, and hydrocephaly. The AFP shows lower sensitivity and specificity in liver cancer at an early stage, and promotes worsening of cirrhosis and chronic hepatitis. Accordingly, some novel markers such as DCP (Des-gamma carboxyprothrombin), PIVKA-II (prothrombin induced by vitamin K absence-II), AFP-L3 (lens cularis agglutinin-reactive), and GPC3 (glypican-3) are currently investigated into whether they can be used as a diagnostic marker for liver cancer at an early stage. Further, another marker for improving diagnosis probability is still needed. Therefore, in order to develop a biological marker for liver cancer at an early stage, the present inventors have analyzed the gene expression profiles for 40 cases of hepatocellular carcinoma tissues and nonhepatocellular carcinoma tissues by a cDNA microarray method, and have selected a gene of secretory protein which is differentially expressed in hepatocellular carcinoma tissues, and then have screened it. They have found that cystatin B (cystatin B, CSTB) can be used as a diagnostic marker for liver cancer at an early stage, thereby completing the present invention.

### [Disclosure]

### [Technical Solution]

Therefore, it is an object of the present invention to provide a method for diagnosing hepatocellular carcinoma comprising the steps of:
contacting a material specific to a CSTB gene or a CSTB protein with a biological sample and
comparing an expression level of the CSTB gene or the CSTB protein in the biological sample with that of a control sample,
wherein the material specific to a CSTB gene is a sense and antisense primer or a probe complementary to mRNA of the CSTB gene and wherein the material specific to the CSTB protein is an antibody specific to the CSTB protein.

It is another object of the present invention to provide a method for determining the progression or prognosis of hepatocellular carcinoma using the method of the invention.

### [Description of Drawings]

Fig. 1 is the result (upper) that mRNA expression of cystatin Bis measured in 30 cases of nonhepatocellular carcinoma tissues (N) and hepatocellular carcinoma tissues (T) by northern blotting, and the histogram (lower) showing that its amount is amplified, as compared to nonhepatocellular carcinoma tissues.
Fig. 2 shows the microarray result of 40 cases of hepatocellular carcinoma tissues and the corresponding nonhepatocellular carcinoma tissues. Fig. 2a is the microarray result showing unsupervised hierarchical clustering analysis of hepatocellular carcinoma tissues and nonhepatocellular carcinoma tissues, and Fig. 2b is a dendrogram showing two categories of nonhepatocellular carcinoma tissues and hepatocellular carcinoma tissues.
Fig. 3 is a photograph of electrophoresis obtained from RT-PCR that was performed using a primer in 15 cases of hepatocellular carcinoma tissues (T), and the corresponding nonhepatocellular carcinoma tissues (N). Fig. 3a is a photograph of electrophoresis obtained from RT-PCR that was performed using primers having SEQ ID NO.: 1 and SEQ ID NO.: 3, and Fig. 3b is a photograph of electrophoresis obtained from RT-PCR that was performed using primers having SEQ. ID. NOs.: 2 and 4.
Fig. 4 is the result of CSTB western blotting using RJMW2E7 monoclonal antibody.
Fig. 5 is the result of immunofluorescence for the position and expression pattern of CSTB in cells.
Fig. 6 is the result of immunochemical staining for CSTB in hepatocellular carcinoma tissues and nonhepatocellular carcinoma tissues by an RJMW2E7 monoclonal antibody. A is normal liver, B is a hepatocellular carcinoma tissue being selectively stained by CSTB antibody, C is a hepatocellular carcinoma tissue, in which CSTB antigen is granulated nucleus or cytoplasm, and D is an invasive hepatocellular carcinoma, in which CSTB is stained in the nucleus and cytoplasm.
Fig. 7 is the values of serum CSTB in healthy persons (G1), patients with noncirrhotic chronic hepatitis (G2), patients with cirrhosis (G3), and patients with hepatocellular carcinoma (G4).
Fig. 8 is a ROC (Receiver operating characteristic) curve for the serum values of CSTB in order to distinguish hepatocellular carcinoma patient group (G4) from noncancerous chronic liver disease (G2+G3).
Fig. 9 is the result of western blotting, in which the expression of CSTB protein is observed in Hep3B cell line, into which recombinant CSTB gene expression vectors (CSTB11, CSTB17, CSTB18, CSTB28) were introduced, and in the control cell, into which empty vectors (VC2, VC4) were introduced.

### [Best Mode]

The present invention relates to a novel diagnostic marker for hepatocellular carcinoma comprising a nucleic acid or protein of cystatin B (CSTB).

The term "diagnosis" as used herein means confirmation of a pathological state. For the purpose of the invention, the diagnosis is to confirm the development of hepatocellular carcinoma by assessing the expression level of the diagnostic marker for hepatocellular carcinoma.

The term "a diagnostic marker, a marker for diagnosis, or a diagnosis marker"as used herein means a material capable of distinguishing hepatocellular carcinoma cells from normal cells, and includes an organic biomolecule such as a polypeptide, a nucleic acid (e.g., mRNA etc.), a lipid, a glycolipid, a glycoprotein, and a sugar (monosaccharide, disaccharide, oligosaccharide etc.), which is expressed at a higher or lower level, as compared to its level in normal cells. The diagnostic marker for hepatocellular carcinoma provided in the present invention is a CSTB gene and a protein thereof, which are each highly expressed in hepatocellular carcinoma cells, as compared to normal cells.

CSTB is a gene encoding protein, liver thiol proteinase inhibitor, and the GeneID of Homo sapiens CSTB is 1476 (NCBI). The cystatin subfamily encompasses proteins that contain multiple cystatin sequences. Some of the members are active cysteine protease inhibitors, while others have lost or perhaps never acquired this inhibitory activity. There are three inhibitory families in the subfamily, including the type 1 cystatin (stefins), type 2 cystatins and kininogens, and the CSTB of the invention is the type 1 cystatin (stefins).

The CSTB gene encodes a stefin that functions as an intracellular thiol protease inhibitor. The protein is able to form a dimer stabilized by noncovalent forces, inhibiting papain and cathepsins 1, h and b. The protein is thought to play a role in protecting against the proteases leaking from lysosomes. Evidence indicates that mutations in this gene are responsible for the primary defects in patients with progressive myoclonic epilepsy. However, the correlation between the protein and hepatocellular carcinoma has has not been well known.

In the present invention, CSTB was found to be used as an early diagnostic marker for hepatocellular carcinoma, in which an increase in CSTB mRNA level of tissue and body fluid and an increase in the antigen protein level of body fluid such as serum and the like, which were obtained from a control group, a chronic hepatitis group, a chronic cirrhosis group, and a hepatocellular carcinoma group, were confirmed.

Described herein is a diagnostic composition for hepatocellular carcinoma comprising a nucleic acid capable of assessing the expression level of the CSTB protein, or a gene encoding the same.

In the invention, the nucleic acid means a material capable of assessing the expression level of the CSTB protein, or a gene encoding the same, preferably the mRNA level of the CSTB gene, and the material includes a pair of a primer or a probe specific to the CSTB gene. The expression of the CSTB protein can be effectively measured at the mRNA level using a pair of a primer or a probe specific to the CSTB gene. Those skilled in the art can design a primer or probe that specifically amplifies the specific region of a gene having a known sequence, in particular a CSTB gene, by NM-1476 (NCBI).

The "primer" herein means a short nucleic acid strand having a free 3-hydroxyl group, which is able to form a base pair with a complementary template, and functions as a starting point for amplifying the template. The primer can initiate DNA synthesis in the presence of a regent for polymerization in a suitable buffer solution, at a suitable temperature (DNA polymerase, or reverse transcriptase) and four different dNTP's (deoxynucleoside triphospates). The primer of the invention is a primer specific to each marker CSTB gene, preferably a sense (forward) and antisense (reverse) strand having a sequence of 7 to 50 nucleotides. The additional applications may be incorporated without changing its property that functions as a starting point for DNA synthesis. Further, the primer sequence of the invention may include a label which can be directly or indirectly detected by spectroscopic, photochemical, biochemical, immunochemical, or chemical means. Examples of the label include enzyme (e.g., horse radish peroxidase, alkaline phosphatase), radioisotope (e.g., ³²P), fluorescent dyes, and chemical group (e.g., biotin). A pair of primer of the invention includes all combinations of primers consisting of forward and reverse primer, preferably a pair of primer giving an analysis result having specificity and sensitivity.

The "probe" in the present invention means a fragment of nucleic acid such as an RNA or DNA, which is several to hundreds of base pairs capable of bonding to mRNA specifically, and is labeled to confirm the presence of specific mRNA. The probe can be prepared in a form of oligonucleotide probe, single stranded DNA probe, double stranded DNA probe, RNA probe or the like.

The primer or probe used in the method of the invention can be chemically synthesized using a phosphoramidite solid support method or other conventional method. The sequence of the nucleic acid can be also modified using the method known in the art. Examples of the modification include, but are not limited to, methylation, capping, substitution with one or more homologue of natural nucleotide, and modification between nucleotides, for example, modification to an uncharged linker (e.g., methyl phosphonate, phosphotriester, phosphoroamidate, and carbamate) or charged linker (e.g., phosphorothioate, phosphorodithioate).

The sequence of target gene was already known, and those skilled in the art can easily determine the various combinations of primer pairs having high specificity and sensitivity, considering a variable such as sequence hybridization in the target gene on the basis of the conventional database. In a specific embodiment of the invention, the expression of CSTB gene was assessed at the mRNA level using primers of SEQ ID NO.: 1, 2, 3, and 4.

In the present invention, examples of the material capable of assessing the expression level of CSTB protein include an "antibody" such as a polyclonal antibody, a monoclonal antibody, and a recombinant antibody, each of which specifically binds to the CSTB protein. As described above, a diagnostic marker protein for liver cancer has been found, whereby those skilled in the art can easily prepare the antibody using the diagnostic marker protein for liver cancer by a known method. The polyclonal antibody can be prepared by a known method in the art, in which the CSTB antigen is injected to an animal, and then the blood is collected from the animal to obtain the serum containing the antibody. The polyclonal antibody can be generated in any animal hosts including goats, rabbits, sheep, monkey, horse, swine, cattle, dog, and the like. The monoclonal antibody can be prepared by a known method in the art such as a hybridoma method (see Kohler and Milstein (1976), European Journal of Immunology 6: 511-519), and a phage antibody library (Clackson et al, Nature, 352: 624-628, 1991; Marks et al, J. Mol. Biol., 222:5 8, 1-597, 1991).

Further, the antibody used in the method of the invention is a complete form having two light chains of full length, and two heavy chains of full length, as well as a functional fragment. The functional fragment of the antibody molecule means a fragment having an antigen binding function at least, such as Fab, F(ab'), F(ab') 2, and Fv.

Also described herein is a diagnostic kit for hepatocellular carcinoma comprising the diagnostic composition for hepatocellular carcinoma capable of assessing the expression level of the CSTB protein, or a gene encoding the same.

The diagnostic kit described herein may further comprise one or more kind of composition, a solution, or an apparatus, which are suitable for the analysis method. The diagnostic kit relates to a kit for detecting diagnostic marker comprising essential factors to perform RT-PCR. The RT-PCR kit may include specific primer pairs for the marker gene, as well as a test tube or a suitable container, a reaction buffer (various pH and concentrations of magnesium), deoxynucleotides (dNTPs), an enzyme such as a Taq-polymerase and a reverse transcriptase, a DNase inhibitor, an RNase inhibitor, DEPC-water, sterilized water, or the like. The RT-PCR kit may further include specific primer pairs for a gene, which is used as a quantitative control. Further, the diagnostic kit may be preferably a DNA chip comprising essential factors for performing a microarray. The DNA chip may include a substrate to which a gene, or a cDNA or oligo-base corresponding to the fragment thereof is attached.

Further, in the case where the material for assessing the protein level is preferably an antibody, the diagnostic kit may be a diagnostic kit comprising essential factors for performing ELISA. The ELISA kit may include a reagent detecting the bound antibody, such as a labeled secondary antibody, a chromophore, an enzyme (e.g., conjugation with an antibody), and a substrate thereof, and also include a specific antibody for the quantitative control protein.

In another embodiment of the invention, a method for diagnosing hepatocellular carcinoma, or a method for determining the progression or prognosis of hepatocellular carcinoma, in which the expression level of the CSTB gene or the CSTB protein is assessed, is provided.

More specifically, the expression level of the gene can be assessed at the level of the mRNA or protein, which can be performed by a known technique to isolate the mRNA or protein from a biological sample using a known method.

The "biological sample" as used herein includes a sample such as tissues, cells, blood, serums, blood plasma, saliva, phlegm, cerebrospinal fluids, or urine, in which the different expression level of the CSTB gene is assessed by occurrence of hepatocellular carcinoma, but are not limited thereto.

Examples of the analysis method to assess the mRNA level include RT-PCR, competitive RT-PCR, real time RT-PCR, RNase protection assay, northern blotting, or DNA chip, but are not limited thereto. The expression level of mRNA in the control and patient with hepatocellular carcinoma or suspected patient with hepatocellular carcinoma can be assessed through the analysis method, and the development of hepatocellular carcinoma can be prognosed by measuring the significant expression level of mRNA, as compared with the control.

In the case of using the antibody to assess the level of the CSTB protein, the CSTB marker protein and an antibody specific thereto in the biological sample form a complex, which is referred as an "antigen-antibody complex" in the invention.

The amount of the antigen-antibody complex can be quantitatively measured by the signal size of a detection label. The detection label can be selected from the group consisting of an enzyme, a fluorescent material, a ligand, a luminescent material, a microparticle, a redox molecule, and a radioisotope, but are not limited thereto.

In a specific embodiment of the invention, the increase in the mRNA expression of the CSTB gene in a tissue was observed by a cDNA microarray method, and northern blot analysis, the CSTB secretion protein in the serum was measured by a sandwich ELISA. Further, the correlation between the increase in CSTB antigen expression and the development of hepatocellular carcinoma was studied by performing immunohistochemistry on the CSTB antigen in the tissue.

The auto-radiogram of northern blot was scanned to measure the signal strength using a LAS3000 system (Fuji photo-film, Japan), in order to make a quantitative and statistical analysis. The expression level of hepatocellular carcinoma tissue was normalized with the expression level of 18S rRNA, based on the nonhepatocellular carcinoma tissue. The CSTB measured value between each group was measured by an unpair-t test or a Mann-Whitney test. With *P* < 0.05, the significant standard was determined. In order to determine the cutoff value of CSTB or AFP, a ROC (receiver operating characteristic) curve analysis was performed using a MedCalc software. The optimal cutoff value of CSTB and AFP having the best diagnostic accuracy was automatically determined at the point of minimum sum of false-positive and false-negative rates by MedCalc program. Each total diagnostic value was represented by the area below the curve (ROC).

The patients can be diagnosed on whether they have hepatocellular carcinoma or not, and the progression or prognosis of hepatocellular carcinoma can be determined, by comparing the expression levels of the gene in normal control with those in suspected patient with hepatocellular carcinoma via the above mentioned analysis method.

Also described herein is a method for screening the CSTB expression inhibitor, which involves comparing the expression levels of the mRNA or protein in a CSTB overexpressed cell line, into which a recombinant expression vector has been introduced, a cell line, which has been treated with a candidate of a CSTB expression inhibitor, and those of a control.

The "recombinant vector" as used herein is a vector capable of expressing a target protein or target RNA in a suitable host cell, and a gene construct containing essential regulatory factors to operate the expression of insertion. The "operably linked" as used herein refers to functional linkage between a regulatory sequence regulating the nucleic acid expression and a nucleic acid sequence encoding the target protein or RNA in order to perform the general functions. For example, a promoter and the nucleic acid sequence encoding the target protein or RNA are operably linked to affect the expression of the nucleic acid sequence encoding the target protein or RNA. The operable linkage with recombinant vector can be prepared using the gene recombinant method known in the art, and the site specific DNA linkage and cleavage are performed with the known enzymes in the art.

Examples of the vector include a plasmid vector, a cosmid vector, a bacteriophage vector, and a virus vector, but are not limited thereto. Examples of the expression vector preferably include regulatory elements such as a promoter, an operator, an initiation codon, a termination codon, a polyadenylation signal, and an enhancer, as well as a signal sequence for membrane targeting or secretion, or a leader sequence, and can be variously prepared according to its purpose. The promoter of the vector may be constitutive or inducible. Further, the expression vector includes a selective marker for selection of a host cell having the vector, and the duplicable expression vector includes the duplication origin.

In the case where the host cell is a bacterium belonging to the genus *Escherichia,* the signal sequence is a PhoA signal sequence, an OmpA signal sequence or the like. In the case where the host cell is a bacterium belonging to the genus *Bacillus,* the signal sequence is an -amylase signal sequence, a subtilisin signal sequence or the like. In the case where the host cell is yeast, the signal sequence is an MF signal sequence, an SUC2 signal sequence or the like. In the case where the host cell is a mammalian cell, the signal sequence is an insulin signal sequence, an -interferon signal sequence, an antibody molecule signal sequence or the like, but is not limited thereto.

A transformation method includes any method for introducing the nucleic acid into a cell, a tissue or an organ of an organism, and can be performed by employing the preferable standard technology according to the host cell as known in the art. Examples thereof include an electroporation, a cytoplasmic fusion, calcium phosphate (CaCl₂), a precipitation, a silicon carbide fiber-mediated transformation, an agrobacterium mediated transformation, PEG, dextran sulfate, and lipofectamine, but are not limited thereto.

The amount of expression and modification are different according to the host cell, and the most preferable host cell is selected and used according to its purpose. Examples of the host cell include a prokaryotic cell such as *Escherichia coli, Bacillus subtilis, Streptomyces, Pseudomonas, Prometeus mirabilis* or *Staphylococcus,* but are not limited thereto. Further, a lower eukaryotic cell such as fungus (e.g., *Aspergillis, Schizosaccharomyces,* and *Neurospora crassa),* and a cell derived from a higher eukaryotic cell including an insect cell, a plant cell, and a mammalian cell can be used as the host cell.

In specific Examples of the invention, a Hep3B cell line overexpressing the CSTB protein, which is transformed by the recombinant vector, is provided. The Hep3B cell line is a human liver cancer cell, which overexpresses the CSTB protein according to the invention. The Hep3B cell line is treated with the CSTB expression inhibitor, and then its expression pattern is observed. As a result, it can be used in screening an anti-cancer agent inhibiting hepatocellular carcinoma.

The method for screening the CSTB expression inhibitor described herein comprises the steps of culturing the cells expressing the CSTB protein, treating the cells with a candidate of CSTB expression inhibitor, and comparing it with the expression level of the diagnostic marker for hepatocellular carcinoma (protein, mRNA) in a control cell, which is not treated with the candidates. The anti-cancer agent, which is capable of inhibiting CSTB expression, and of effectively treating liver cancer, can be searched using the screening method of the invention.

As a screening method, a method for measuring the expression level of the CSTB gene, preferably mRNA level using primer or probe can be used. Among them, a RT-PCR method is preferred. The inhibitor capable of reducing the expression of the diagnostic marker, which compared with control cell, is screened to be used in treatment of hepatocellular carcinoma.

Also described herein is a preventive or therapeutic composition for hepatocellular carcinoma comprising the CSTB expression inhibitor. Specifically described herein is a preventive or therapeutic composition for hepatocellular carcinoma comprising the CSTB expression inhibitor including a complementary antisense RNA and a complementary sense RNA strand to the mRNA of the CSTB gene, and comprises siRNA inducing RNA interference, which is specific to the CSTB gene, and.a preventive or therapeutic composition for hepatocellular carcinoma comprising antibodies, which are specific to the CSTB protein.

The term "siRNA" as used herein means a double stranded RNA capable of inducing RNA interference (RNAi) via mRNA cleavage of the target gene, which consists of a sense RNA strand having homologous sequence with mRNA of the target gene and an antisense RNA strand having complementary sequence to the sense RNA strand. The siRNA can inhibit the expression of the target gene, which can be provided as a gene knockdown method or gene therapy.

The siRNA is not limited to the region of double stranded RNA completely pairing with RNAs, and may include the region of double stranded RNA not completely pairing by the presence of mismatch (the corresponding base is not complementary), bulge (the corresponding base is not present in one strand) or the like. The total length thereof is 10 to 80 base pairs, preferably 15 to 60 base pairs, more preferably 20 to 40 base pairs. The structure of the ends of siRNA can be a blunt end or a cohesive end. The cohesive end may have a protruding end at 3 terminus and 5 terminus, and the number of the protruding base is not limited. For example, the number of the base can be 1 to 8 bases, preferably 2 to 6 bases. Further, the siRNA may include, in the range of maintaining the effect inhibiting the target gene expression, for example low molecular RNA (e.g., natural RNA such as tRNA, rRNA, virus RNA or artificial RNA) at the protruding end of one terminus. The structure of the ends of siRNA is not need to have cleavage structures at both ends, and may be a stem loop type structure that one end of the double stranded RNA is connected by a linker RNA. The length of the linker is not limited, as long as the length does not hamper base pairing of the stem. The method for preparing siRNA is a method that siRNA is synthesized in vitro, and then transfected into the cells, and a method that a siRNA expression vector expressing siRNA in the cells or a PCR-derived siRNA expression cassette is transferred into the cells by gene delivery or transduction.

The term "specific" as used herein means the ability to inhibit only the target gene without affecting other genes in the cells, and the siRNA is specific to the CSTB in the present invention.

The siRNA of the present disclosure can reduce the mRNA of CSTB specifically, and its sequence and length are not limited. The composition comprising the siRNA specific to the gene may include an additional material inhibiting cell death, and include a preparation promoting the entrance of siRNA into the cells. The preparation promoting the entrance of siRNA into the cells can generally use a preparation promoting the entrance of nucleic acid. For example, it can use liposome and be mixed with a lipophilic carrier selected from sterols such as cholesterol, cholate, and deoxycholate. Further, a cationic polymer such as poly-L-lysine, spermine, polysilazane, polyethylenimine (PEI), polydihydroimidazolenium, polyallylamine, and chitosan can be used, and an anionic polymer such as succinylated PLL, succinylated PEI, polyglutamic acid, polyaspartic acid, polyacrylic acid, polymethacylic acid, dextran sulfate, heparin, and hyaluronic acid can be used.

If the antibody specific to the CSTB protein is used as a therapeutic antibody, the antibody can be coupled (e.g., covalently bonded) with a conventional therapeutic agent directly,or indirectly via a linker. Examples of the therapeutic agent capable of coupling with the antibody include a radionuclide, a drug, a lymphokine, a toxin, a heterofunctional antibody, but are not limited thereto. (1) a radionuclide such as 131I, 90Y, 105Rh, 47Sc, 67Cu, 212Bi, 211At, 67Ga, 125I, 186Re, 188Re, 177Lu, 153Sm, 123I, and 111In, (2) a biological modification or drug including lymphokine such as methotrexate, adriamycin, and interferon, (3) a toxin such as ricin, abrin, and diphtheria, (4) a heterofunctional antibody, an antibody that binds to other antibody to form a complex, that is, a complex capable of binding to both of cancerous cells and effector cells (e.g., a killer cell such as a T cell), and (5) a natural, nonassociated or noncomplexed antibody.

The antibody, as it is, or a composition comprising the antibody can be administered.

The therapeutic composition can be prepared with a pharmaceutically acceptable carrier according to its administration route. The preferred administration route is well known, and a surfactant facilitating membrane transport is included. The surfactant can be induced from steroid or can be a cationic lipid such as N-[1-(2,3-dioleoil(propyl)-N,N,N-trimethylammoniumchloride (DOTMA), or various compounds such as cholesterol hemisuccinate and phosphatidyl glycerol.

The composition comprising the antibody of the present disclosure can be administered in a pharmaceutically effective amount to treat cancerous cells or their metastasis.

The pharmaceutical composition can be administered singly or in combinations. The composition comprising the antibody is administered via a subcutaneous, intraperitoneal, intrapulmonary, or intranasal route, if necessary for immune suppressive treatment, by any suitable method including lesion readministration. Examples of the parenteral injection include intramuscular, intravenous, intraarterial, intraperitoneal, or subcutaneous administrations. The preferred administration route and preparation is an intravenous, subcutaneous, intracutaneous, intramuscular, or drop preparation. The pH of preparation can adjust the antibody stability (chemical and physical stability) to modify other suitable technology for administration, thereby being able to design another suitable preparation. The typical amount level for administration can be optimized using a standard method. Further, the antibody of the invention is administered in the form of a nucleic acid encoding the antibody, so as to generate the antibody in the cells (WO96/07321).

### [Mode for Invention]

Hereinafter, the present invention will be described in more detail with reference to Examples. However, these Examples are for the illustrative purpose only, and the invention is not intended to be limited by these Examples.

### (Example 1)Isolation of total RNA from hepatocellular carcinoma tissue and nonhepatocellular carcinoma tissue

Hepatocellular carcinoma tissues and surrounding nonhepatocellular carcinoma tissues were removed from 40 patients with hepatocellular carcinoma by a surgical operation to perform the study, and the present study was approved by Research Ethics Committee at Chonbuk National University. The 40 patients with hepatocellular carcinoma underwent the surgical operation for curative treatment, and the carcinoma tissue and the surrounding cirrhotic tissue were obtained from the removed liver tissue. Paper agreements were taken and the hepatocellular carcinoma tissue and the nonhepatocellular carcinoma tissue were histologically confirmed by a pathologist. After removing the tissues, the tissues were washed with a sterilized phosphate buffer, and then stored in a nitrogen tank for further isolating total RNAs. The total RNAs were extracted with a Trizol Kit (MRC Co., USA), and the quality was confirmed using electrophoresis. Some of the removed tissues were fixed in a 10% formalin buffer, and embedded in paraffin to perform standard histopathological analysis. The protocol was performed in accordance with the Code of Ethics of Research Ethics Committee, and the total RNAs were extracted from the hepatocellular carcinoma tissues and the surrounding nonhepatocellular carcinoma tissues using a Trisol solution, respectively.

### (Example 2)Gene profiling of hepatocellular carcinoma

The total RNAs of the hepatocellular carcinoma tissues and the nonhepatocellular carcinoma tissues extracted in Example 1 were hybridized with about 3000 genes on the cDNA microarray, and then fluorescently labeled cDNA was prepared to divide two main groups by unsupervised hierarchical clustering analysis. A gene to discriminate between nonhepatocellular carcinoma tissues and hepatocellular carcinoma tissues was identified in 1% or less of FDR (false discovery rate) by a statistical program.

The total RNAs from the hepatocellular carcinoma tissues and the surrounding nonhepatocellular carcinoma tissues were extracted using the Trisol solution, and then fluorescently labeled cDNA was prepared to hybridize with the microarray. 100 µg of the isolated total RNAs were dissolved in 14 µl, and then SuperSript II transcriptase, 0.5 mM of dATP, dGTP, dCTP, and 0.2 mM of dTTP were added thereto. 0.1 mM of Cy5-dUTP or Cy3-dUTP was added thereto to perform labeling in 40 µl of the final volume for 2 hours at 42C, and then 5 µl of 500 mM EDTA was added to terminate the labeling. 10 µl of 1N NaOH was added to the RNA being not labeled, so as to hydrolyze for 30 minutes at 65C. 25 µl of Tris HCL (pH 7.5) was added thereto, and neutralized to remove unlabeled bases and salts using Biospin 6 column. The labeled probes were precipitated with isopropanol, and then dissolved in a hybridization buffer. The dissolved probes were put on a slide, and then covered with a hybri-slip to place in a hybridization chamber at 65C overnight for reaction. After hybridization, the slides were washed with 1 SSC/0.1% SDS, 0.1 SSC/0.1% SDS (50), and 0.1 SSC buffer for 10 minutes, respectively. The terminated slides were scanned using the Quantarray program and analyzed using the ImaGene 4.2, Biodiscovery program.

The fluorescent strength of Cy5 and Cy3 took local background correction using the ImaGene program, to normalize using total spots. The qualitative analysis of the spots was performed using the following criterion. The spots passing the criterion in which the mean signal is 1.5 times more than local background are used in analysis, among the spots of which value of Signal mean-Background mean/Background is SD > 2.0 or more. The criterion was identically applied to all array slide analysis.

Cancer classification was analyzed with Cluster and TreeView available on website. The array factors that can be measured at 80% or more in the used samples were used for the analysis. Before analysis, the fluorescence ratios of each spot were adjusted by log-transformation, and a median centering was preformed in order to remove experimental biases. Statistical analysis was performed using SAM (Significance analysis of microarray) program, and the significant genes were scored. The genes, which were expressed 80% or more in the hepatocellular carcinoma tissues and the nonhepatocellular carcinoma tissues, were screened to try hierarchical clustering analysis for all of the tissues, based on the similarity to the expression pattern of total genes.

All of the tissues were divided into a major cancer and non-cancer tissue cluster, and only one case of the nonhepatocellular carcinoma tissues belonged to the hepatocellular carcinoma cluster. 248 genes were overexpressed and 149 genes were underexpressed in the hepatocellular carcinoma tissues, as compared with the nonhepatocellular carcinoma tissues. Among them, top 20 genes, which are differentially expressed in the hepatocellular carcinoma and the nonhepatocellular carcinoma, are listed in Tables 1 and 2.

**[Table 1]**

| Gene ID | Annotation | Symbol | Mean fold change | p-Value | q-Value |
|---|---|---|---|---|---|
| Hs.44532 | Ubiquitin D | UBD | 2.5851522 | 1.00E-04 | 0.00016 |
| Hs.3136 | Protein kinase, AMP-activated, gamma 1 non-catalytic subunit | PRKAG1 | 2.2441856 | 1.00E-04 | 0.00016 |
| Hs.695 | Cystatin (stefin B) | CSTB | 2.1175641 | 1.00E-04 | 0.00016 |
| Hs.507 | Psoriasis susceptibility 1 candidate 1 | PRORS1C1 | 2.0031053 | 1.00E-04 | 0.00016 |
| Hs.11083 | Tubulin, beta, 5 | TUBB5 | 1.9962115 | 1.00E-04 | 0.00016 |
| Hs.62914 | Transcribed sequences | | 1.981338 | 1.00E-04 | 0.00016 |
| Hs.3459 | Similar to Ubiquitin binding protein | UBPH | 1.9463814 | 1.00E-04 | 0.00016 |
| Hs.111779 | Secreted protein, acidic, cysteine-rich (osteonectin) | SPARC | 1.8742631 | 1.00E-04 | 0.00016 |
| Hs.161357 | Pyruvate dehydrogenase (lipoamide) beta | PDHB | 1.8638526 | 1.00E-04 | 0.00016 |
| Hs.93379 | Eukayotic translation initiation factor 4B | EIF4B | 1.8629581 | 1.00E-04 | 0.00016 |
| Hs.1710 | ATP-binding cassette, sub-family B (MDR/TAP), member 10 | ABCB10 | 1.8350107 | 1.00E-04 | 0.00016 |
| Hs.30340 | Nedd4 family interacting protein 2 | NDFIP2 | 1.8060788 | 1.00E-04 | 0.00016 |
| Hs.90436 | Sperm associated antigen 7 | SPAG7 | 1.8000763 | 0.0078 | 0.00661 |
| Hs.10842 | RAN, member RAS oncogene family | RAN | 1.7669291 | 1.00E-04 | 0.00016 |
| Hs.111244 | DNA-damage-inducible transcript | DDIT4 | 1.7596031 | 1.00E-04 | 0.00016 |
| Hs.8102 | Ribosomal protein S20 | RPS20 | 1.7239815 | 1.00E-04 | 0.00016 |
| Hs.62595 | Chromosome 9 open reading frame | C9orf9 | 1.6816082 | 2.00E-04 | 0.000285 |
| Hs.72242 | TBC1 domain family, member 14 | TBC1D14 | 1.6555126 | 1.00E-04 | 0.00016 |
| Hs.108966 | Phosphatidylinositol-4-phosphate 5-kinase, type II, alpha | PIP5K2A | 1.64066334 | 1.00E-04 | 0.00016 |
| Hs.157607 | Sorting nexin 22 | SNX22 | 1.6269205 | 1.00E-04 | 0.00016 |

**[Table 2]**

| Gene ID | Annotation | Symbol | Mean fold change | p-Value | q-Value |
|---|---|---|---|---|---|
| Hs.1290 | Complement component 9 | C9 | 6.157514 | 1.00E-04 | 0.00016 |
| Hs.75183 | Cytochrome P450, family 2, subfamily E, polypeptide 1 | CYP2E1 | - 3.580571 | 1.00E-04 | 0.00016 |
| Hs.85155 | Zinc finger protein 36, C3H type-like 1 | ZFP36L1 | - 3.015888 | 1.00E-04 | 0.00016 |
| Hs.1282 | Complement component 6 | C6 | 2.956622 | 1.00E-04 | 0.00016 |
| Hs.80756 | Hetaine-homocysteine methyltransferase | BHMT | - 2.846807 | 1.00E-04 | 0.00016 |
| Hs.165551 | Flavoprotein oxidoreductase MICAL3 | MICAL3 | - 2.786589 | 1.00E-04 | 0.00016 |
| Hs.24583 | Hypothetical protein DKFZp434C0328 | DKFZp434C0328 | - 2.739747 | 1.00E-04 | 0.00016 |
| Hs.1051 | Granzyme B (granzyme 2, cytotoxic T-lymphocyte-associated serine esterase 1) | GZMB | 2.701592 | 1.00E-04 | 0.00016 |
| Hs.1872 | Phosphoenolpyruvate carboxykinase 1 (soluble) | PCK1 | -2.6855 | 1.00E-04 | 0.00016 |
| Hs.10319 | UDP glycosyltransferase 2 family, polypeptide 37 | UGT2B7 | -2.5277 | 1.00E-04 | 0.00016 |
| Hs.132230 | Similar to fibronectin type 3 and SPRY domain-containinq protein | MGC45564 | 2.382785 | 1.00E-04 | 0.00016 |
| Hs.75275 | Ubiquitination factor E4A (UFD2 homolog, yeast) | UBE4A | - 2.336673 | 1.00E-04 | 0.00016 |
| Hs.92025 | KIAA0316 gene product | KIAA0316 | 2.310326 | 4.00E-04 | 0.000504 |
| Hs.2523 | Alcohol dehydrogenase 1C (class I), gamma polypeptide | ADH1C | - 2.295308 | 1. OOE-04 | 0.00016 |
| Hs.139876 | Ribosomal protein S9 | RPS9 | 2.259602 | 1.00E-04 | 0.00016 |
| Hs.135742 | Sideroflexin 1 | SFXN1 | 2.256822 | 1.00E-04 | 0.00016 |
| Hs.36793 | Solute carrier family 12 (potassium/chloride transporters), member 8 | ' SLC12A8 | -2.21657 | 1.00E-04 | 0.00016 |
| Hs.93199 | Apolipoprotein A-I | APOA1 | - 2.180906 | 1.00E- | 0.00016 |
| Hs.69771 | B-factor, properdin | BF | - 2.155754 | 1. 00E-04 | 0.00016 |
| Hs.37 | Acetyl-coenzyme A acetyltransferasel (acetoacetyl Coenzyme A thiolase) | ACAT1 | - 2.148807 | 1.00E-04 | 0.00016 |

It is a critical diagnostic value that CSTB, a gene encoding a secretory protein, are selectively overexpressed in the hepatocellular carcinoma tissues. Therefore, it was seen that the expression of CSTB RNA and protein in a biological sample including tissue and body fluid was assessed, thereby using as a diagnostic and prognostic index for hepatocellular carcinoma.

### (Example 3)Analysis of CSTB expression in nonhepatocellular carcinoma group corresponding to hepatocellular carcinoma group

### (1) Northern blot analysis

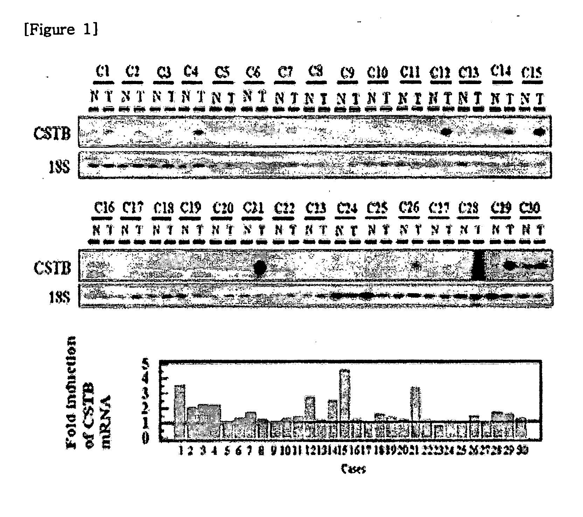

A sample containing 20 ug of total RNAs from hepatocellular carcinoma and nonhepatocellular carcinoma was loaded into 1% agarose containing 2.2% formaldehyde and 50 mM 3-(*N*-morpholino)propanesulfonic acid (MOPS) and then transferred to a nylon membrane to treat with UV cross-linker (stratagem Co., USA). The blot was hybridized with 2 X 10⁶ cpm/ml of CSTB cDNA probe labeled with [³²P]dCTP(NEN) by random-priming overnight, and then washed to expose to a X-Omat AR film (Kodak) at -70C. The blot was stripped and rehybridized with the cDNA of 18S ribosomal protein gene as a loading control. It was found that CSTB mRNA was differentially expressed in hepatocellular carcinoma tissues, as compared to nonhepatocellular carcinoma tissues by the northern blot (80%) (Fig. 1). The membrane for northern blot was washed and rehybridized with 18S cDNA as a probe to use for comparative loading test, and then the relative expression of each cancerous tissue to noncancerous tissue was normalized with the expression of 18S to determine. The bottom histogram of Fig. 1 shows that the increased level of CSTB mRNA in the hepatocellular carcinoma was normalized with the expression amount of 18S mRNA to quantify in the ratio to the nonhepatocellular carcinoma, in which in 24 cases of 30 cases (80%), the level of CSTB mRNA in the hepatocellular carcinoma increased one or more times than that in the nonhepatocellular carcinoma.

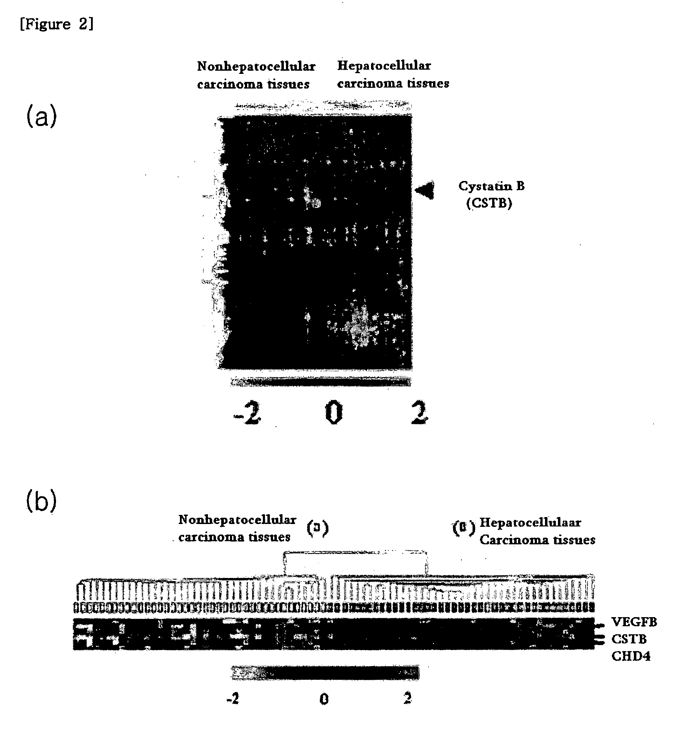

Fig. 2a shows that the microarray result of the hepatocellular carcinoma tissues from 40 patients and the corresponding nonhepatocellular carcinoma tissues was analyzed with unsupervised hierarchical clustering. The columns represent each gene, and the rows represent the patients. The red color represents that the gene was overexpressed, the green color represents that the gene was underexpressed, and the scale is a logarithmic scale based on log2. The dendrogram in Fig. 2b shows two categories, that is, nonhepatocellular carcinoma and hepatocellular carcinoma. Most regions of CSTB gene in the hepatocellular carcinoma tissue were observed with the red color. Therefore, CSTB was found to be overexpressed in the hepatocellular carcinoma tissues.

### (2) Amplification of cDNA using reverse transcriptase polymerase chain reaction (RT-PCR)

Total RNAs were extracted with phenol and GTC solution (guanidine thiocyanate solution, Tri Reagent; Molecular Research Center, Inc. Cincinnati, OH) from the tissues or the cultured cell lines. 40 u/*µ*ℓ of RNAse inhibitor was added thereto to synthesize cDNA. 5 fold-volume of reverse transcriptase buffer, 8 *µ*ℓ oligo decamer primer (500 ng/ml 4 µℓ), 3.2 *µ*ℓ of 250 µM dNTP and 2 *µ*ℓ of superscript RTase (200 U/*µ*ℓ) were added to the tRNA precipitate, 37°C and subjected to reaction in 40 *µ*ℓ of total reaction volume at 37°C for 50 minutes to prepare cDNA. On the basis of the cDNA, 2 *µ*ℓ of cDNA, each 10 µM of forward primer

(5'-GTCGCCGCCAAGATGATGTGC-3'; SEQ ID NO.: 1 or 5'-TGTCATTCAAGAGCCAGGTG-3'; SEQ ID NO.: 2) and reverse primer (5'-GAAATAGGTCAGCTCATCATG-3'; SEQ ID NO.: 3 or 5'-GCTCTGGTAGACGGAGGATG-3'; SEQ ID NO.: 4) containing 5' untranslated region of CSTB mRNA were added thereto, and mixed with 4 *µ*ℓ of 2.5 mM dNTP, 5 *µ*ℓ of ten-fold reverse transcriptase buffer, 3 *µ*ℓ of 10 mM MgCl₂, 0.5 *µ*ℓ of Taq polymerase to be 50 °C of total reaction volume, and then the reaction was terminated. At this time, denaturation was performed at 94 °C for 3 minutes, and 35 cycles of denaturation (94°C for 1 minute), annealing (50°C for 1 minute) and extension (72°C for 1 minute) were performed to complete the reaction. The PCR products were resolved on 2% agarose gels.

### (3) Expression analysis of CSTB mRNA by detection primer for gene expression (SEQ ID NO.: 1 and 3, or SEQ ID NO.: 2 and 4)

The mRNAs were extracted from the hepatocellular carcinoma tissues and the corresponding nonhepatocellular carcinoma tissues to synthesize cDNA using random octamer oligonucleotide as a primer under the reverse transcriptase. PCR was performed with the cDNA as a template, forward and reverse primers, and Taq polymerase.

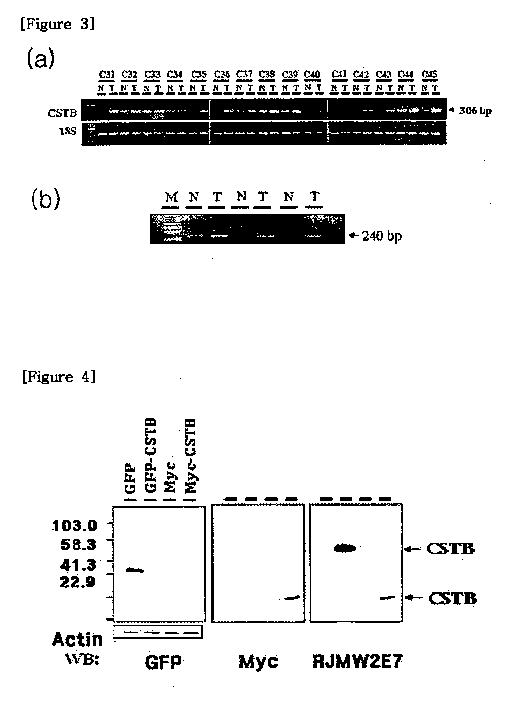

As the PCR result by the forward primer containing 5 untranslated region of CSTB mRNA with SEQ ID NO.: 1 and 3 and the reverse primer containing translated region, the mRNA was selectively expressed only in the cancerous tissues (T). Thus, a band of 306 bases was observed (Fig. 3a). CSTB mRNA was found to be overexpressed in 12 cases of 15 hepatocellular carcinoma cases (80%). As the PCR result by the forward primer and reverse primer containing translated region of CSTB mRNA with SEQ ID NO.: 2 and 4, a band of 240 bases was observed (Fig. 3b).

### (4) Immunoblot immunofluorescent staining and immunochemical staining

The protein was extracted with 1% Triton X-100 containing aprotnin (1 trypsin unit/ml), leupeptin (10 µg/ml), and pepstatin A (10 µg/ml) from the cell or serum, and then SDS-PAGE was performed. The electrophoresed gel was transferred to a nitrocellulose membrane, and nonspecific binding was blocked with 5% dried skim milk in a Tween 20 tris buffer. Anti human CSTB antibody, as a primary antibody, was added to the membrane, and left at room temperature for 1 hour. Then, secondary antibody binding with HRPenzyme was added thereto, and left at room temperature for 1 hour. A luminescent agent, ECL (Amersham Bioscience Co.) was added to detect a signal of protein band (Henikoff S, Gene(Amst) 1984; 28: 351-59). An RJMW2E7 monoclonal antibody (LBS Co.) was used for detecting the CSTB protein. In order to determine the immunoreactive specificity of antibodies to the CSTB protein, GFP-labeled CSTB or Myc-labeled CSTB expression vector was introduced to 293T cell lines, and immunoblot was performed to analyze the immunoreactivity of the antibodies. The introduced CSTB cDNA was labeled with GFP or Myc protein at the C-terminus, and then subjected to immunoblot with anti-GFP antibody (Santa Cruz, FL, USA) or anti-Myc antibody (9E10, Santa Cruz). A mouse monoclonal antibody, RJMW2E7 was found to specifically detect a band of CSTB (Fig. 4). The arrows represent the CSTB protein, and the monoclonal antibody, RJMW2E7 was found to specifically bind the GFP or Myc-labeled CSTB protein.

Alternatively, as an immunofluorescent staining method, the GFP-labeled CSTB expression vector (pEGFP-C2-CSTB) and the control empty vector (pEGFP-C2) were introduced to Hep3B and 293T cell lines, so as to determine the immunoreactivity of the monoclonal antibody, RJMW2E7. For immunofluorescent staining, the cells were cultured on coverslips, and introduced with a GFP-labeled CSTB plasmid. The control test was performed only with the vector. The cells were fixed in 4% paraformaldehyde to induce transparency with phosphate buffer containing 0.2% triton, and then blocked with 1% BSA. Then, the cells were left in the mouse monoclonal antibody, RJMW2EM, and further left in TRITC (fluorescence-labeled anti-mouse antibody). After final washing, the nuclei of the cells were stained in 1mg/ml Hoechst 33258 for 15 minutes, mounted in 50% glycerol, and observed in scan microscopy (LCM510 Zeiss, Germany).

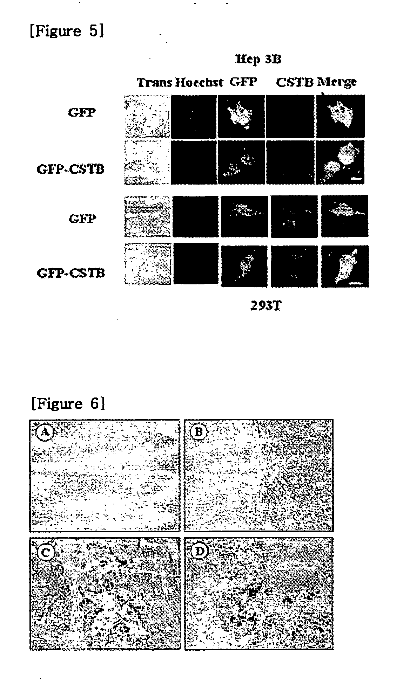

From the comparison of fluorescent and transparent image, the exogenous expression of GFP-labeled CSTBis shown in the nucleus or cytoplasm, which is identical to a prior report, and completely overlapped with immunoreactivity of red fluorescent CSTB. The endogenous expression of GFP-labeled CSTBis found to be mainly shown in the cytoplasm by RJMW2E7 (Fig. 5). The immunoreactivity of GFP protein and CSTB protein is completely overlapped. As a result, it can be seen that the RJKMW2E7 antibody has specificity.

Next, the paraffin-embedded CSTB expression was analyzed in the nonhepatocellular carcinoma tissue corresponding to the hepatocellular carcinoma using the RJMW2E7 antibody.

Immunohistochemistry was performed with an immuno streptavidin-biotin-peroxidase reagent (Biomedia, Foster City, CA). The normal and hepatocellular carcinoma tissues were fixed in 10% formalin and embedded in paraffin, which is the general method for treating a pathological tissue. The paraffin was removed from the tissue with xylene, and then washed with 1 M tris buffer to inactivate the endogenous peroxidase with methanol periodic acid at 40°C for 2 minutes. The tissue sections were left in the primary CSTB monoclonal antibody at 40°C for 9 minutes, and then left biotin-anti rabbit immunoglobulin at 40°C for 4 minutes. Then, the tissue sections were developed with 3-amino-9-ethylcabazole, 0.05% hydrogen peroxide, and a buffer of pH 5.2. The negative control was stained with a physiological saline buffer or mouse IgG1 immunoglobulin instead of the primary antibody.

(A) in Fig. 6 shows a CSTB negative normal liver including hepatic cell, blood vessel, and bile duct, and (B) shows that the hepatocellular carcinoma tissue is selectively and strongly stained by the CSTB antibody, as compared to the nonhepatocellular carcinoma tissue. (c) is CSTB positive hepatocellular carcinoma tissue showing that CSTB antigen protein is granulated in the nucleus or cytoplasm, and (D) is invasive hepatocellular carcinoma showing CSTB staining in the nucleus or cytoplasm. That is, the CSTB is selectively expressed in the hepatocellular carcinoma tissue. It was found that the positive responses were shown in the cytoplasm of most hepatocellular carcinoma tissues, and rarely shown in the cytoplasm and nucleus.

### (Example 4) measurement of serum CSTB value according to type of liver disease and its progression.

Based on demographic and clinical data, sera were collected from 4 groups, in which a group 1 (G1) is a healthy person group having normal biochemical signs and no history of liver disease, a group 2 (G2) is a patient group having noncirrhotic chronic hepatitis, which is histologically confirmed, group 3 (G3) is cirrhosis patient group having compensated and uncompensated liver disease, which is histologically and clinically confirmed, and group 4 (G4) is hepatocellular carcinoma patient group, which is histologically confirmed.

The cause of underlying liver disease is based on hepatitis B, in which serum hepatitis B surface antigen is positive, hepatitis C, in which serum hepatitis C virus antibody and viral RNA were detected, and alcoholic liver disease, in which the patients have drunken daily 40 g or more for at least 15 years. The liver disease, of which the cause is not clear, is classified into a cryptogenic liver disease. The sera were centrifuged, and then immediately stored at -20°C.

The serum CSTB may be topically and systemically expressed in the form of secretory protein in accordance with the expression pattern of cancerous tissue. Therefore, the serum CSTB values were measured in 52 cases of G1 group, 53 cases of G2 group, 43 cases of G3 group, and 62 cases of G4 group using a sandwich ELISA method. The result is a mean value of two individual measurements, and the statistical significance of the mean values between each group was analyzed by the Mann-Whitney test. The significance was determined with P < 0.05. The four groups are classified according to sex, age, and cause. The age of G2 to G4 is 43.7 11.8 (mean SD), 54.1 10.1, and 59.1 ± 10.4 (P < 0.016), which means that hepatocellular carcinoma occurs at the terminal stage of chronic liver disease, and the difference of MELD score is not obvious between G3 group and G4 group (12.8 ±5.3:12.4 ±6.3).

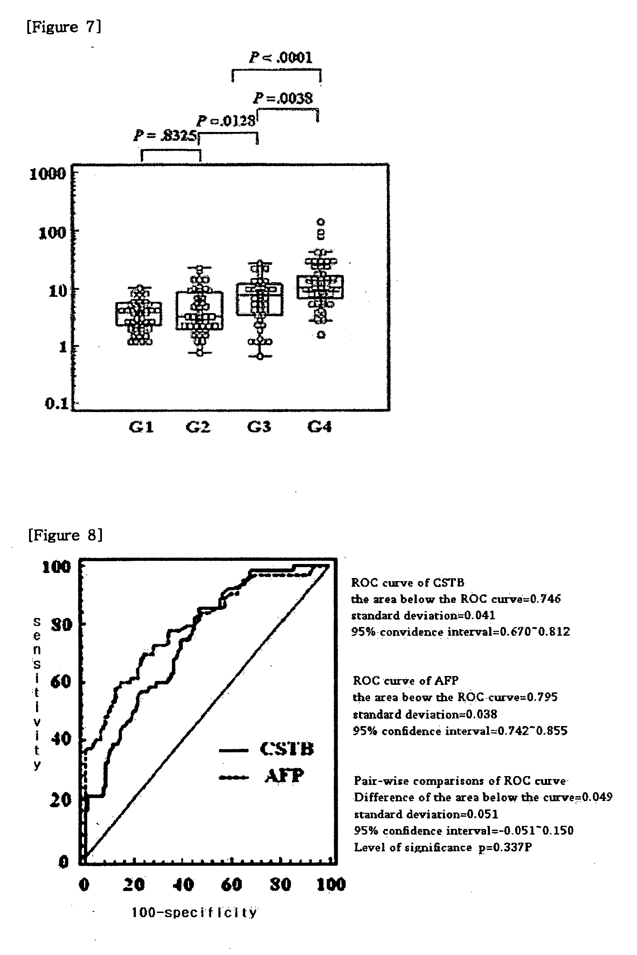

The CSTB values of G2 group, G3 group, and G4 group were 3.3, 7.8, and 10.8ng/ml (P < 0.001), respectively, and the values significantly increased. The difference between G1 group and G2 group was not obvious (P=0.8325). Each CSTB value of the G3 group and G4 group significantly increased, as compared to G2 group (P=0.0128 and P < 0.001), and the G2 group had higher CSTB value than G3 group (P=0.0038). The CSTB value in hepatocellular carcinoma tissue significantly increased, as compared to nonmalignant liver disease, G2+G3. (P<0.001) (Fig. 7).

In Fig. 8, the serum values of CSTB were comparatively shown by ROC (Receiver operating characteristic) curve in order to distinguish G4, hepatocellular carcinoma patient group from G2+G3.

From the comparison of serum values between CSTB of the invention and alpha fetal protein (AFP), each desirable value was 5.34 ng/ml and 32.6 ng/ml. The sensitivity and specificity of CSTB were 84.4% (95%CI, 73.1%-92.2%) and 53.1% (15%CI, 42.7%-63.4%), and those of AFP were 56.7% (95%CI, 43.3%-68.8%) and 87, 5% (95%CI, 79.2%-93.4%), respectively. The positive predictive value and negative predictive value of CSTB were 54.5 and 83.6, and those of AFP were 75 and 76.4. In the area under the ROC curves (AUC), the difference of sensitivity and specificity between CSTB and AFPwas not obvious (0.741:0.782, P=0.429). In this result, the desirable value of CSTB was shown to have higher sensitivity and lower specificity than that of AFP. In order of determine the sensitivity of CSTB value according to the size of tumor mass for early diagnosis of hepatocellular carcinoma, the frequency of hepatocellular carcinoma showing higher CSTB value than desirable value was compared with the case of showing higher AFP value than desirable value (Table 3).

In the hepatocellular carcinoma tissue with the size of 3 cm or less, the positive CSTB showed 73.3% of sensitivity, and in the hepatocellular carcinoma tissue with the size of 3 cm or more, the positive CSTB showed 89.4% of sensitivity. On the contrary, the AFP values showed 60% and 57.4% of frequency, respectively. It can be said that CSTB shows higher sensitivity in diagnosis of hepatocellular carcinoma or progressive hepatocellular carcinoma than AFP.

**[Table 3]**

| Tumor size (cm) | CSTB ) 5.34 ng/ml | AFP ) 32.6 ng/ml | *P* |
|---|---|---|---|
| ( 3 | 73.3% (11/15) | 60% (9/15) | 0.700 |
| ≥ 3 | 89.4% (42/47) | 57.4% (27/47) | ( 0.001 |
| Total | 85.5%(53/62) | 58.1% (36/62) | 0.001 |

### (Example 5) Preparation of CSTB recombinant gene and establishment of cell line overexpressing CSTB recombinant gene

Human hepatoma Hip 3B cell line was purchased from ATCC (USA), and cultured at 37°C under 5% CO₂. Essential amino acids and Pyruvate were added to MEM medium containing 10% FBS to use. The Myc-labeled CSTB was prepared using a pCDNA3.1/Myc vector (Invitrogen, USA) to introduce into the cells, and an empty vector (pCDNA3.1/Myc-HisA vector) was introduced into the cells as a control.

Human CSTB gene was obtained from 21C Frontier Human Gene Bank of Korea Research Institute of Bioscience and Biotechnology, and cDNA encoding the CSTB protein was amplified using the gene as a template, a forward primer (SEQ ID NO.: 1) Having EcoRI restriction site, and a reverse primer (SEQ ID NO.: 3) Having XhoI restriction site by PCR, and then digested with restriction enzyme, EcoRI and Xho1 to perform electrophoresis on a gel. Then, a band was cut, and extracted using a gene extraction kit (Accurse gel purification kit; Pioneer, Daejeon, Korea) to use as an insert. A vector pcDNA3.1/Myc-HisA was digested with EcoRI and SalI to perform electrophoresis on a gel in the same manner. A band was cut and extracted using the gene extraction kit (Accurse gel purification kit; Pioneer, Daejeon, Korea) to use as a vector.

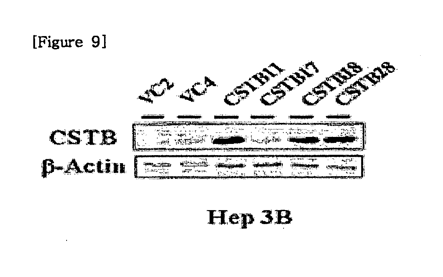

The insert was inserted into the vector frame using a ligation kit, cloned, and introduced into Elcoli to amplify. Thus, the prepared plasmid was purified. When the hepatoma Hip 3B cells were cultured to 70% of 6 cm dish, the gene was transfected using a lipofectamine (Gibco, Invitrogen Co.). After 48 hours, the cells were cultured in the selective medium containing G418 for 2 to 3 weeks. Each formed colony was placed 6-well plate, and then transferred to T25 flask to observe its morphology. The control cell line at 2 weeks and cell line-overexpressing CSTB at 4 weeks were separated and the overexpression of CSTB protein was confirmed by CSTB immunoblot (Fig. 9).

After pCDNA3.1/Myc-HisA-CASTB vector was introduced, CSTB overexpression was observed in CSTB 11,17,18,28, which is established. CSTB 11,17,18,28 were more expressed than VC2 and VC4 control cell lines expressing the empty vector (pCDNA3.1/Myc-HisA vector). The present cell lines can be a material for further studying the effect of the CSTB protein expression on hepatocellular carcinoma cell line, and used in the molecular biological target studies for diagnosis and treatment of hepatocellular carcinoma.

### [Industrial Applicability]

As described above, the present invention provides a cystatin B (CSTB) gene as a diagnostic marker for hepatocellular carcinoma, in which the CSTB gene expression as a tumor marker is fast and sensitively quantitated in patient's tissue, and the CSTB protein expression as a product of the gene is effectively detected in body fluid, thereby being used in early-diagnosis of hepatocellular carcinoma and diagnosis of progressive, invasive, and metastatic hepatocellular carcinomas.
## Claims

1. A method for diagnosing hepatocellular carcinoma comprising the steps of:
contacting a material specific to a CSTB gene or a CSTB protein with a biological sample and
comparing an expression level of the CSTB gene or the CSTB protein in the biological sample with that of a control sample,
wherein the material specific to a CSTB gene is a sense and antisense primer or a probe complementary to mRNA of the CSTB gene and wherein the material specific to the CSTB protein is an antibody specific to the CSTB protein.

2. The method according to claim 1, wherein the step of contacting the material specific to the CSTB gene is performed by an RT-PCR, a competitive RT-PCR, a real time RT-PCR, an RNase protection assay, a northern blotting or a DNA chip.

3. A method for determining progression or prognosis of hepatocellular carcinoma, using the method according to claim 1 or 2.

## Patentansprüche

1. Verfahren zur Diagnostizierung eines hepatozellulären Karzinoms, das die folgenden Schritte umfasst:
In-Kontakt-Bringen eines Materials, das spezifisch für ein CSTB-Gen oder ein CSTB-Protein ist, mit einer biologischen Probe und
Vergleichen eines Expressionsniveaus des CSTB-Gens oder des CSTB-Proteins in der biologischen Probe mit dem einer Kontrollprobe,
wobei das Material, das spezifisch für ein CSTB-Gen ist, ein Sense- und Antisense-Primer oder eine Sonde ist, der bzw. die komplementär zu der mRNA des CSTB-Gens ist, und wobei das Material, das spezifisch für das CSTB-Protein ist, ein Antikörper ist, der spezifisch für das CSTB-Protein ist.

2. Verfahren nach Anspruch 1, wobei der Schritt des In-Kontakt-Bringens des Materials, das spezifisch für das CSTB-Gen ist, durch eine RT-PCR, eine kompetitive RT-PCR, eine Echtzeit-RT-PCR, ein RNase-Schutz-Assay, ein Northern Blotting oder einen DNA-Chip durchgeführt wird.

3. Verfahren zur Bestimmung der Progression oder der Prognose eines hepatozellulären Karzinoms, das das Verfahren nach Anspruch 1 oder 2 verwendet.

## Revendications

1. Procédé pour diagnostiquer un carcinome hépatocellulaire comprenant les étapes consistant à :
mettre en contact un matériau spécifique d'un gène CSTB ou d'une protéine CSTB avec un échantillon biologique et
comparer un niveau d'expression du gène CSTB ou de la protéine CSTB dans l'échantillon biologique avec celui d'un échantillon de contrôle,
dans lequel le matériau spécifique à un gène CSTB est une amorce sens et anti-sens ou une sonde complémentaire d'un ARNm du gène CSTB et dans lequel le matériau spécifique de la protéine CSTB est un anticorps spécifique de la protéine CSTB.

2. Procédé selon la revendication 1, dans lequel l'étape consistant à mettre en contact le matériau spécifique au gène CSTB est réalisée par une RT-PCR, une RT-PCR compétitive, une RT-PCR en temps réel, un essai de protection à la RNase, un buvardage de Northern ou une puce ADN.

3. Procédé pour déterminer la progression ou le pronostic d'un carcinome hépatocellulaire en utilisant le procédé selon la revendication 1 ou 2.
